# EUROPÄISCHE PATENTSCHRIFT

(11) **EP 1 367 057 B1**
(45) Veröffentlichungstag und Bekanntmachung des Hinweises auf die Patenterteilung: **17.09.2008**
(21) Anmeldenummer: 03016552.6
(22) Anmeldetag: 18.11.1997
(51) Int. Cl.: C07D 417/06, C07D 493/04, A01N 43/90, C12P 17/16, A61K 31/427, A61P 35/00

(54) **Epothilone E und F**
Epothilones E and F
Epothilones E et F

(30) Priorität: 18.11.1996 DE 19647580; 25.02.1997 DE 19707506
(43) Veröffentlichungstag der Anmeldung: 03.12.2003
(62) Teilanmeldung aus: 97951233.2
(73) Patentinhaber: Gesellschaft für Biotechnologische Forschung mbH (GBF), 38124 Braunschweig (DE)
(72) Erfinder: Reichenbach, Hans, D-38124 Braunschweig (DE); Höfle, Gerhard, D-38124 Braunschweig (DE); Gerth, Klaus, D-38124 Braunschweig (DE); Steinmetz, Heinrich, D-38124 Braunschweig (DE)
(74) Vertreter: Boeters, Hans Dietrich

(56) Entgegenhaltungen:
- WO-A-93/10121
- WO-A-97/19086
- WO-A-98/08849
- BALOG A. ET AL.: "Total synthesis of (-)-epothilone A" ANGEWANDTE CHEMIE, INTERNATIONAL EDITION IN ENGLISH, Bd. 35, Nr. 23/24, 3. Januar 1997 (1997-01-03), Seiten 2801-2803, XP002035359
- NICOLAOU K.C. ET AL.: "An approach to epothilones based on olefin metathesis" ANGEWANDTE CHEMIE, INTERNATIONAL EDITION IN ENGLISH, Bd. 35, Nr. 20, 4. November 1996 (1996-11-04), Seiten 2399-2401, XP002035372

## Beschreibung

Die vorliegende Erfindung betrifft Epothilone E und F sowie ein therapeutisches Mittel.

So betrifft die Erfindung eine Verbindung (Epothilon E) der Formel:

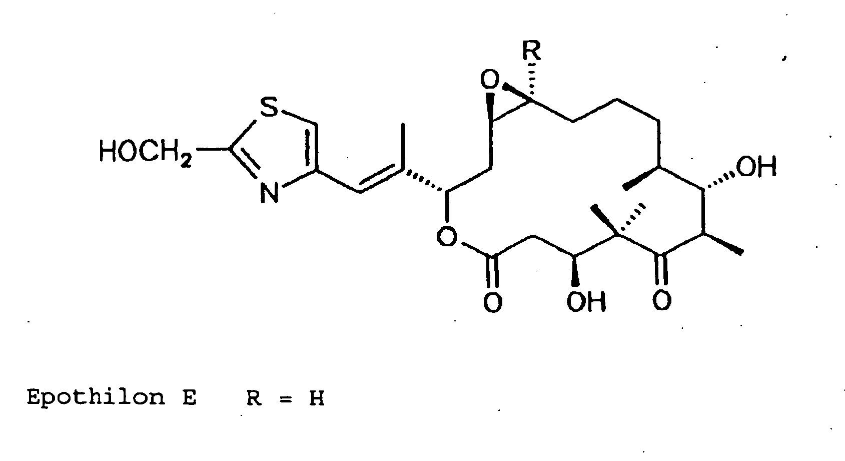

Ferner betrifft die Erfindung eine Verbindung der Summenformel C₂₆H₃₉NO₇S, gekennzeichnet durch folgendes ¹H-NMR-Spektrum (300 MHz, CDCl₃): delta = 2.38 (2-Hₐ), 2.51 (2-H_{b}), 4.17 (3-H), 3.19 (6-H), 3.74 (7-H), 1.30 - 1.70 (8-H, 9-H₂, 10-H₂, 11-H₂), 2.89 (12-H), 3.00 (13-H), 1.88 (14-Hₐ) , 2.07 (14-H_{b}) , 5.40 (15-H), 6.57 (17-H), 7.08 (19-H), 4.85 (21-H₂), 1.05 (22-H₃), 1.32 (23-H₃), 1.17 (24-H₃), 0.97 (25-H₃), 2.04 (27-H₃)

Ferner betrifft die Erfindung eine Verbindung (Epothilon F) der Formel:

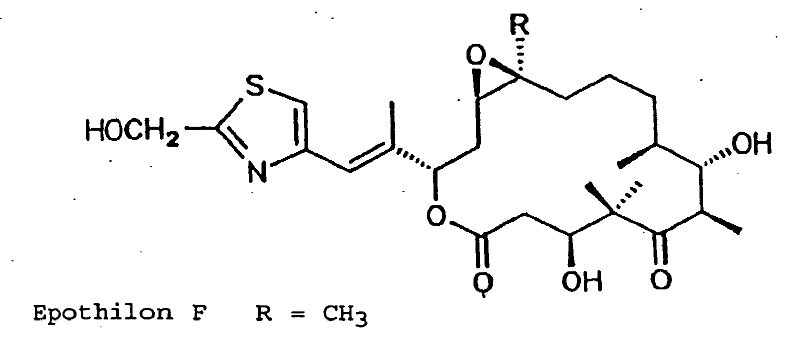

Ferner betrifft die Erfindung eine Verbindung der Summenformel C₂₇H₄₁NO₇S, gekennzeichnet durch folgendes ¹H-NMR-Spektrum (300 MH_{z}, CDCl₃): delta = 2.37 (2-Hₐ), 2.52 (2-H_{b}), 4.20 (3-H), 3.27 (6-H), 3.74 (7-H), 1.30- 1.70 (8-H, 9-H₂, 10-H₂, 11-H₂), 2.78 (13-H), 1.91 (14-H), 2.06 (14-H_{b}), 5.42 (15-H), 6.58 (17-H), 7.10 (19-H), 4.89 (21-H₂), 1.05 (22-H₃), 1.26 (23-H₃), 1.14 (24-H₃), 0.98 (25-H₃), 1.35 (26-H₃), 2.06 (27-H₃).

Schließlich betrifft die Erfindung ein therapeutische Mittel, bestehend aus einer oder mehreren der vorstehend aufgeführten Verbindungen oder bestehend aus einer oder mehreren der vorstehend aufgeführten Verbindungen neben einem oder mehreren üblichen Träger(n) und/oder Verdünnungsmittel(n). Dieses Mittel kann insbesondere cytotoxische Aktivitäten zeigen und/oder Immunsuppression bewirken und/oder zur Bekämpfung maligner Tumore eingesetzt werden, wobei es besonders bevorzugt als Cytostatika verwendbar ist.

Die Erfindung wird im folgenden durch die Beschreibung von einigen ausgewählten Ausführungsbeispielen näher erläutert und beschrieben.

### Beispiele

### Beispiel 1:

### Epothilon E und F, neue Biotransformationsprodukte der Epothilone A und B.

### Produktionsstamm:

Der Produktionsstamm *Sorangium cellulosum* So ce90 wurde im Juli 1985 an der GBF aus einer Bodenprobe von den Ufern des Zambesi isoliert und am 28.10.91 bei der Deutschen Sammlung für Mikroorganismen unter Nr. DSM 6773 hinterlegt.

Die Charakterisierung des Produzenten sowie die Kulturbedingungen sind beschrieben in:
Höfle, G.; N. Bedorf, K. Gerth & H. Reichenbach: Epothilone, deren Herstellungsverfahren sowie sie enthaltende Mittel. DE 41 38 042 A1, offengelegt am 27. Mai 1993.

### Bildung der Epothilone E und F während der Fermentation:

Eine typische Fermentation verläuft folgendermaßen: Ein 100 l Bioreaktor wird mit 60 l Medium (0,8 % Stärke; 0,2 % Glucose; 0,2 % Soyamehl; 0,2 % Hefeextrakt; 0,1 % CaCl₂ x 2H₂O; 0,1 % MgSO₄ x 7H₂O; 8 mg/l Fe-EDTA; pH 7,4) gefüllt. Zusätzlich werden 2 % Adsorberharz (XAD-16, Rohm & Haas) zugegeben. Das Medium wird durch Autoklavieren (2 Std., 120 °C) sterilisiert. Beimpft wird mit 10 l einer im gleichen Medium (zusätzlich 50 mM HEPES-Puffer pH 7,4) im Schüttelkolben angezogenen Vorkultur (160 Upm, 30 °C). Fermentiert wird bei 32 °C mit einer Rührergeschwindigkeit von 500 Upm und einer Belüftung von 0,2 Nl pro m³ und Std, der pH Wert wird durch Zugabe von KOH bei 7,4 gehalten. Die Fermentation dauert 7 bis 10 Tage. Die gebildeten Epothilone werden während der Fermentation kontinuierlich an das Adsorberharz gebunden. Nach Abtrennen der Kulturbrühe (z. B. durch Absieben in einem Prozeßfilter) wird das Harz mit 3 Bettvolumen Wasser gewaschen und mit 4 Bettvolumen Methanol eluiert. Das Eluat wird zur Trockne eingeengt und in 700 ml Methanol aufgenommen.

### HPLC-Analyse des XAD-Eluates:

Gegenüber dem Ausgangsvolumen des Reaktors (70 l) ist das Eluat 100:1 konzentriert. Die Analyse wird durchgeführt mit einer HPLC Anlage 1090 der Fa. Hewlett Packard. Zur Trennung der Inhaltstoffe wird eine Microbore Säule (125/2 Nucleosil 120-5 C₁₈) der Fa. Machery-Nagel (Düren) verwendet. Eluiert wird mit einem Gradienten aus Wasser/Acetonitril von anfänglich 75:25 bis zu 50:50 nach 5,5 Minuten. Dieses Verhältnis wird bis zur 7. Minute gehalten, um dann bis zur 10. Minute auf 100 % Acetonitril anzusteigen.

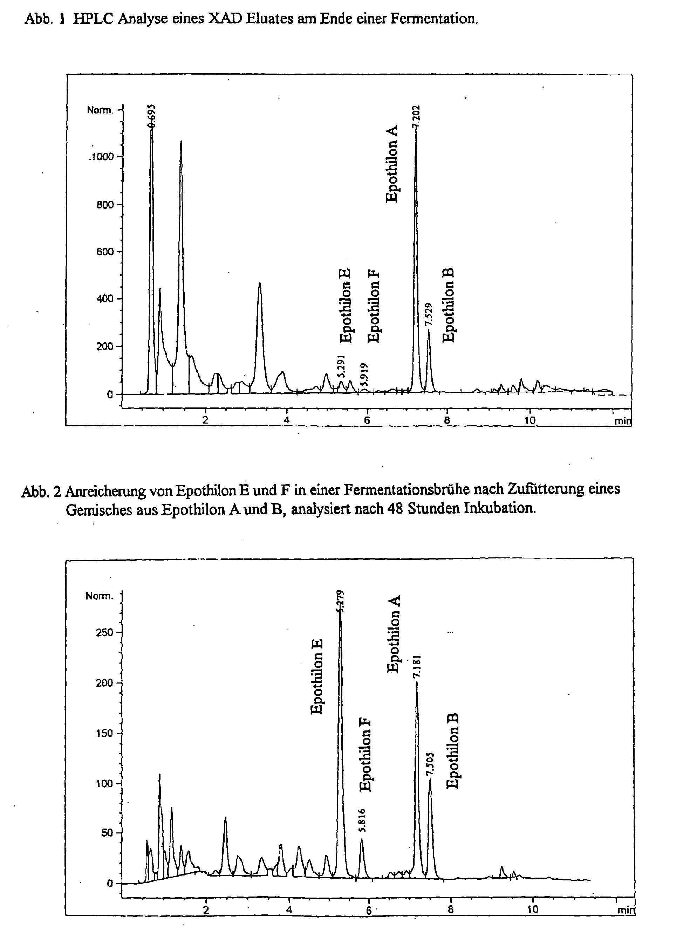

Gemessen wird bei einer Wellenlänge von 250 nm und einer Bandbreite von 4 nm. Die Dioden Array Spektren werden im Wellenlängenbereich von 200 bis 400 nm gemessen. Im XAD-Eluat fallen zwei neue Substanzen mit Rₜ 5,29 und Rₜ 5,91 auf, deren Adsorptionsspektren mit denen von Epothilonen A bzw. B identisch sind (Abb. 1; E entspricht A, F entspricht B). Diese Substanzen werden unter den gegebenen Fermentationsbedingungen nur in Spuren gebildet.

### Biotransformation von Epothilon A und B zu Epothilon E und F:

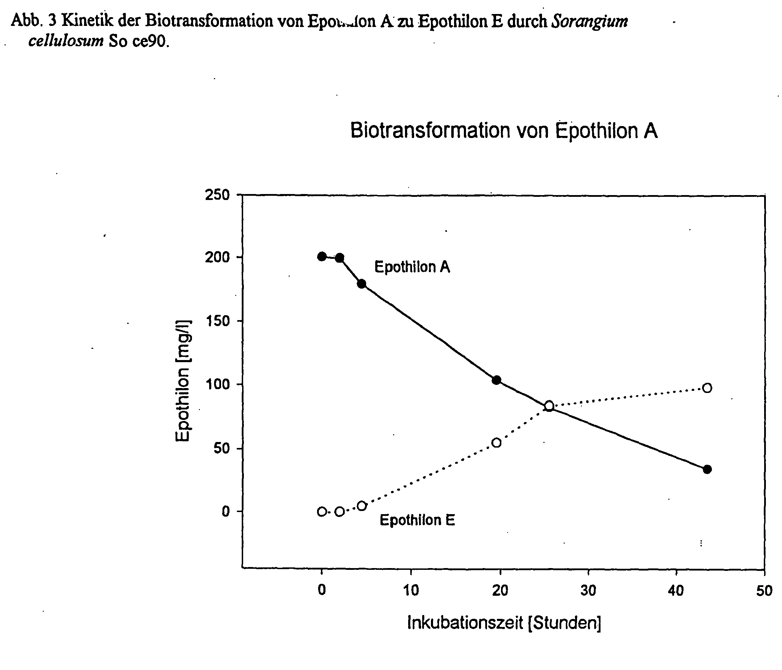

Für die gezielte Biotransformation wird eine 4 Tage alte, mit Adsorberharz gehaltene 500 ml Kultur von So ce90 verwendet. Von dieser werden 250 ml unter Zurücklassen des XAD in einen sterilen 1 l Erlenmeyerkolben überführt. Danach wird eine methanolische Lösung einer Mischung von insgesamt 36 mg Epothilon A und 14 mg Epothilon B zugegeben und der Kolben für zwei Tage bei 30 °C und 200 Upm auf einer Schütteltruhe inkubiert. Die Bildung der Epothilone E und F wird direkt aus 10 µl des zentrifugierten Kulturüberstands analysiert (**Abb. 2**). Die Umwandlung erfolgt nur in Gegenwart der Zellen und ist abhängig von der eingesetzten Zelldichte und der Zeit. Eine Kinetik der Umwandlung ist für Epothilon A in **Abb.** 3 dargestellt.

### Isolierung von Epothilon E und F

Zur Isolierung von Epothilon E und F werden drei Schüttelkolbenansätze aus der Biotransformation (s. o.) vereinigt und 1 h mit 20 ml XAD-16 geschüttelt. Das XAD wird durch Absieben gewonnen und mit 200 ml Methanol eluiert. Das Eluat wird i. Vak. zu 1.7 g Rohextrakt eingedampft. Dieser wird zwischen 30 ml Ethylacetat und 100 ml Wasser verteilt. Aus der Ethylacetatphase werden beim Eindampfen i. Vak. 330 mg eines öligen Rückstandes erhalten, die in fünf Läufen über eine 250 x 20 mm RP-18 Säule chromatographiert werden (Laufmittel: Methanol/Wasser 58:42, Detektion 254 nm).

| | |
|---|---|
| Ausbeute: Epothilon | E 50 mg |
| | F 10 mg |

### Biologische Wirkung von Epothilon E:

In Zellkulturen wurde die Konzentration bestimmt, welche das Wachstum um 50 % reduziert (IC₅₀) und mit den Werten für Epothilon A verglichen.

| Zellinie | IC₅₀ (ng/ml) | |
|---|---|---|
| | Epothilon E | Epothilon A |
| HeLa. KB-3.1 (human) | 5 | 1 |
| Mausfibroblasten, L929 | 20 | 4 |

### Epothilon E

C₂₆H₃₉HO₇S [509]
ESI-MS: (positiv Ionen): 510.3 für [M+H]⁺

DC: R_{f} = 0,58
DC-Alufolie 60 F 254 Merck. Laufmittel: Dichlormethan/Methanol = 9:1
Detektion: UV-Löschung bei 254 nm. Ansprühen mit Vanillin-Schwefelsäure-Reagenz, blau-graue Anfärbung beim Erhitzen auf 120 °C
   HPLC: Rₜ = 5,0 min
   Säule: Nucleosil 100 C-18 7µm, 250 x 4 mm
   Laufmittel: Methanol/Wasser = 60:40
   Fluß: 1,2 ml/min
   Detektion: Diodenarray
¹H-NMR (300 MHz, CDCl₃): delta = 2.38 (2-Hₐ) , 2.51 (2-H_{b}), 4.17 (3-H), 3.19 (6-H), 3.74 (7-H), 1.30 - 1.70 (8-H, 9-H₂, 10-H₂, 11-H₂), 2.89 (12-H), 3.00 (13-H), 1.88 (14-Hₐ), 2.07 (14-H_{b}), 5.40 (15-H), 6.57 (17-H), 7.08 (19-H), 4.85 (21-H₂), 1.05 (22-H₃), 1.32 (23-H₃), 1.17 (24-H₃), 0.97 (25-H₃), 2.04 (27-H₃)

### Epothilon F

C₂₇H₄₁NO₇S [523]
ESI-MS: (positiv Ionen): 524.5 für [M+H]⁺

DC: R_{f} = 0,58
DC-Alufolie 60 F 254 Merck. Laufmittel: Dichlormethan/Methanol = 9:1
Detektion: UV-Löschung bei 254 nm. Ansprühen mit Vanillin-Schwefelsäure-Reagenz, blau-graue Anfärbung beim Erhitzen auf 120 °C.
   HPLC: Rₜ = 5,4 min
   Säule: Nucleosil 100 C-18 7µm, 250 x 4 mm
   Laufmittel: Methanol/Wasser = 60:40
   Fluß: 1,2 ml/min
   Detektion: Diodenarray
¹H-NMR (300 MH_{z}, CDCl₃): delta = 2.37 (2-Hₐ), 2.52 (2-H_{b}), 4.20 (3-H), 3.27 (6-H), 3.74 (7-H), 1.30 - 1.70 (8-H, 9-H₂, 10-H₂, 11-H₂), 2.78 (13-H), 1.91 (14-H), 2.06 (14-H_{b}), 5.42 (15-H), 6.58 (17-H), 7.10 (19-H), 4.89 (21-H₂), 1.05 (22-H₃), 1.26 (23-H₃), 1.14 (24-H₃), 0.98 (25-H₃), 1.35 (26-H₃), 2.06 (27-H₃).

### Beispiel 2:

### Herstellung von Epothilon E und F durch Biotransformation mit Sorangium cellulosum So ce90

### 1) Durchführung der Biotransformation:

Für die Biotransformation wird eine Kultur von *Sorangium cellulosum* So ce90 verwendet, die für vier Tage in Gegenwart von 2 % XAD 16 Adsorberharz (Fa. Rohm und Haas, Frankfurt/M.) bei 30 °C und 160 Upm geschüttelt wurde. Das Kulturmedium hat folgende Zusammensetzung in g/Liter destilliertem Wasser: Kartoffelstärke (Maizena), 8; Glucose (Maizena), 8; entfettetes Sojamehl, 2; Hefeextrakt (Marcor), 2; Ethylendiamintetraessigsäure, Eisen (III) Natrium Salz, 0,008; MgSO₄ x 7 H₂O, 1; CaCl₂ x 2 H₂O, 1; HEPES 11,5. Der pH-Wert wird vor dem Autoklavieren mit KOH auf 7,4 eingestellt. Das XAD wird durch Sieben über ein Edelstahlsieb (200 µm Maschenweite) von der Kultur abgetrennt. Die Bakterien werden durch Zentrifugation für 10 min bei 10 000 Upm sedimentiert und das Pellet in 1/5 des Kulturüberstandes resuspendiert. Zu der konzentrierten Bakteriensuspension wird nun Epothilon A bzw. Epothilon B in methanolischer Lösung in einer Konzentration von 0,5 g/Liter zugesetzt. Die Kultur wird wie oben beschrieben weiterkultiviert. Zur Analyse der Biotransformation wird zu den gewünschten Zeiten eine 1 ml Probe entnommen, 0,1 ml XAD zugegeben und die Probe für 30 min bei 30 °C geschüttelt. Eluiert wird das XAD mit Methanol. Das Eluat wird zur Trockene eingeengt und in 0,2 ml Methanol wieder aufgenommen. Diese Probe wird über HPLC analysiert.

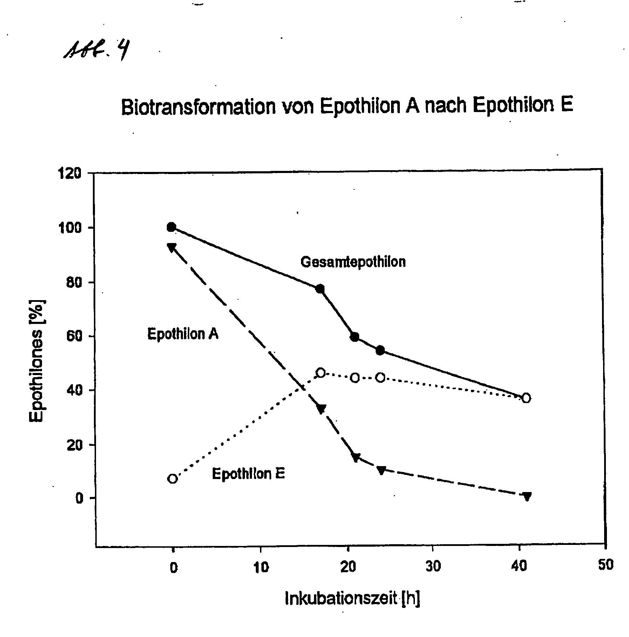

Abb. 4) Kinetik der Biotransformation von Epothilon A nach Epothilon E

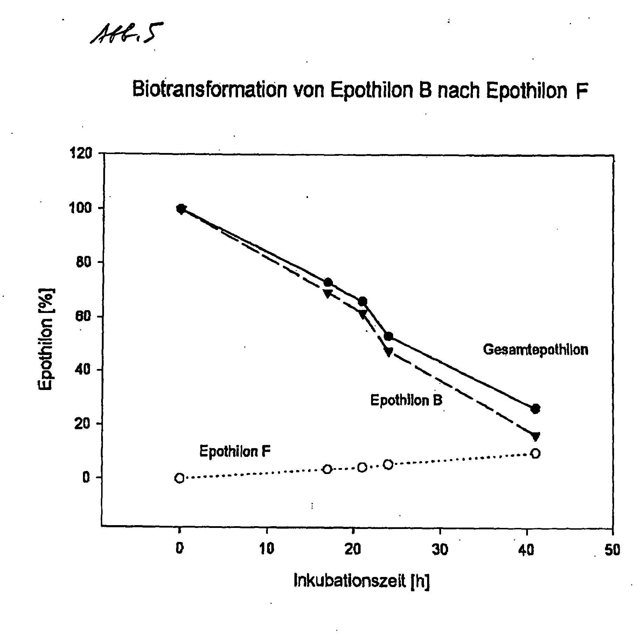

Abb. 5) Kinetik der Biotransformation von Epothilon B nach Epothilon F.

### 2) Herstellung von Epothilon E durch Biotransformation von 1 g Epothilon A.

Der Stamm *Sorangium cellulosum* So ce90 wird für vier Tage in 8,5 1 des obigen Mediums (jedoch ohne XAD Zusatz) in einem 10 Liter Bioreaktor bei 30 °C, einer Drehzahl von 150 Upm und einer Belüftung von 0,1 vvm angezogen.

Anschließend wird die Kultur durch cross flow Filtration auf 3 l eingeengt. Hierzu werden 0,6 m² einer Membran mit einer Porengröße von 0,3 µm verwendet.

Die konzentrierte Kultur wird in einen 4 Liter Bioreaktor überführt und eine methanolische Lösung von 1 g Epothilon A in 10 ml Methanol zugegeben. Anschließend wird die Kultur über einen Zeitraum von 21,5 h weiterkultiviert. Die Temperatur beträgt 32 °C, die Rührerdrehzahl 455 Upm und die Belüftung erfolgt mit 6 l/min. Zum Erntezeitpunkt wird 100 ml XAD zugegeben und für 1 h weiterinkubiert. Das XAD wird durch Absieben von den Zellen abgetrennt und erschöpfend mit Methanol eluiert. Das konzentrierte Eluat wird über HPLC analysiert.

### Bilanzierung der Biotransformation:

| | |
|---|---|
| Epothilon A eingesetzt | 1000 mg = 100 % |
| Epothilon A nach 21,5 h wiedergefunden | 53,7 mg = 5,4 % |
| Epothilon E nach 21,5 h gebildet | 661,4 mg = 66,1 % |
| Epothilon A vollständig abgebaut | = 28,5 % |

### Beispiel 3:

Die erfindungsgemäßen Epothilone E und F wurden mit Zellkulturen (Tabelle 1) und auf Polymerisationsförderung (Tabelle 2) getestet.

**Tabelle 1: Epothilon-Tests mit Zellkulturen**

| **Epothilon** | **A** **493** | **B** **507** **IC-50** | **C** **477** **[ng/ml]** | **D** **491** | **E** **509** | **F** **523** |
|---|---|---|---|---|---|---|
| Mausfibroblasten L 929 | 4 | 1 | 100 | 20 | 20 | 1,5 |
| | | | | | | |
| humane Tumorzellinien: | | | | | | |
| HL-60 (Leukämie) | 0.2 | 0.2 | 10 | 3 | 1 | 0,3 |
| K-562 (Leukämie) | 0.3 | 0.3 | 20 | 10 | 2 | 0,5 |
| U-937 (Lymphom) | 0.2 | 0.2 | 10 | 3 | 1 | 0,2 |
| KB-3.1 (Cervixkarzinom) | 1 | 0.6 | 20 | 12 | 5 | 0,5 |
| KB-V1 (Cervixkarzinom multires | 0.3 | 0.3 | 15 | 3 | 5 | 0,6 |
| A-498 (Nierenkarzinom) | - | 1.5 | 150 | 20 | 20 | 3 |
| A-549 (Lungenkarzinom) | 0.7 | 0.1 | 30 | 10 | 3 | 0,1 |

**Tabelle 2: Polymerisationstest mit Epothilonen**

| Parameter: Zeit bis zur halbmaximalen Polymerisation der Kontrolle | | | | | | |
|---|---|---|---|---|---|---|
| Messung: | w | x | y | z | **Mittel** | **Mittel** |
| | | | | | **[s]** | **[%]** |
| Kontrolle | 200 | 170 | 180 | 210 | **190** | **100** |
| Epothilon A | 95 | 60 | 70 | 70 | **74** | **39** |
| Epothilon B | | 23 | 25 | 30 | **26** | **14** |
| Epothilon C | 125 | 76 | 95 | 80 | **94** | **49** |
| Epothilon D | 125 | 73 | 120 | | **106** | **56** |
| Epothilon E | 80 | 60 | 50 | 45 | **59** | **31** |
| Epothilon F | 80 | 40 | 30 | 50 | **50** | **26** |

### Standardtest mit 0,9 mg Tubulin/ml und 1 µM Probenkonzentration

Der Polymerisationstest ist ein in vitro Test mit gereinigtem Tubulin aus Schweinehirn. Die Auswertung erfolgt photometrisch. Polymerisationsfördernde Substanzen wie die Epothilone verkürzen die Zeit, bis zu der halbmaximale Polymerisation erfolgt ist, d. h., je kürzer die Zeit, desto wirksamer die Verbindung. w, x, y und z sind vier unabhängige Versuche, die relative Wirksamkeit ist in der letzten Spalte in % der Kontrolle ausgedrückt; wieder zeigen die niedrigsten Werte die beste Wirksamkeit an. Die Rangliste entspricht ziemlich genau der in Zellkulturen festgestellten.

## Patentansprüche

1. Verbindung (Epothilon E) der Formel:

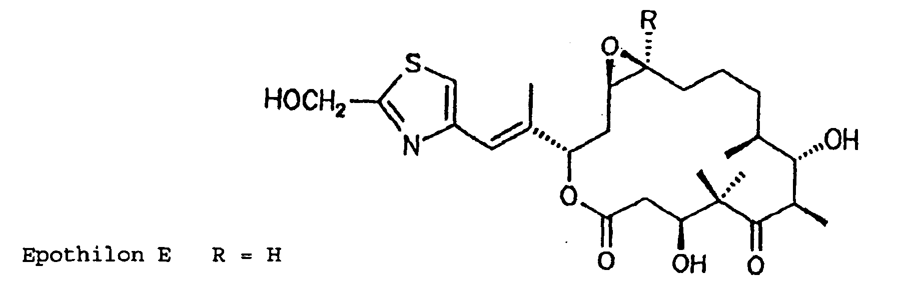

2. Verbindung der Summenformel C₂₆H₃₉NO₇S nach Anspruch 1, **gekennzeichnet durch** folgendes ¹H-NMR-Spektrum (300 MHz, CDCl₃): delta = 2.38 (2-Hₐ), 2.51 (2-H_{b}), 4.17 (3-H), 3.19 (6-H), 3.74 (7-H), 1.30 - 1.70 (8-H, 9-H₂, 10-H₂, 11-H₂), 2.89 (12-H), 3.00 (13-H), 1.88 (14-Hₐ), 2.07 (14-H_{b}), 5.40 (15-H), 6.57 (17-H), 7.08 (19-H), 4.85 (21-H₂), 1.05 (22-H₃), 1.32 (23-H₃), 1.17 (24-H₃), 0.97 (25-H₃), 2.04 (27-H₃).

3. Verbindung (Epothilon F) der Formel:

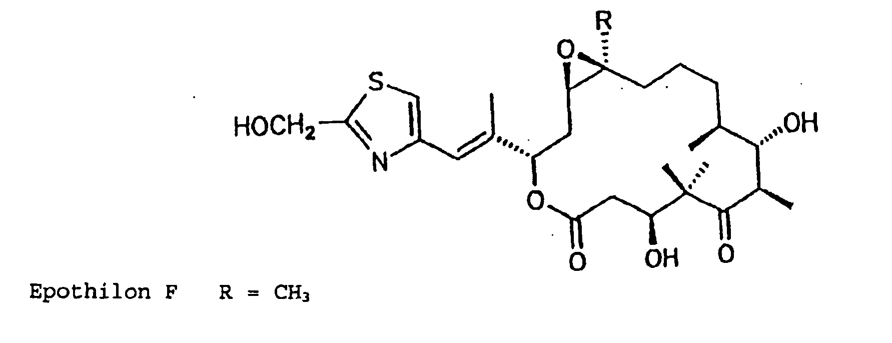

4. Verbindung der Summenformel C₂₇H₄₁NO₇S nach Anspruch 3, **gekennzeichnet durch** folgendes ¹H-NMR-Spektrum (300 MH_{z}, CDCl₃): delta = 2.37 (2-Hₐ) , 2.52 (2-H_{b}), 4.20 (3-H) , 3.27 (6-H) , 3.74 (7-H), 1.30 - 1.70 (8-H, 9-H₂, 10-H₂, 11-H₂), 2.78 (13-H), 1.91 (14-H), 2.06 (14-H_{b}), 5.42 (15-H), 6.58 (17-H), 7.10 (19-H), 4.89 (21-H₂), 1.05 (22-H₃), 1.26 (23-H₃), 1.14 (24-H₃), 0.98 (25-H₃), 1-35 (26-H₃), 2.06 (27-H₃).

5. Therapeutisches Mittel, insbesondere zum Einsatz als Cytostatikum, bestehend aus einer oder mehreren der Verbindungen nach einem oder mehreren der vorhergehenden Ansprüche oder bestehend aus einer oder mehreren der Verbindungen nach einem oder mehreren der vorhergehenden Ansprüche neben einem oder mehreren üblichen Träger(n) und/oder Verdünnungsmittel(n).

## Claims

1. Compound (epothilone E) of the formula:

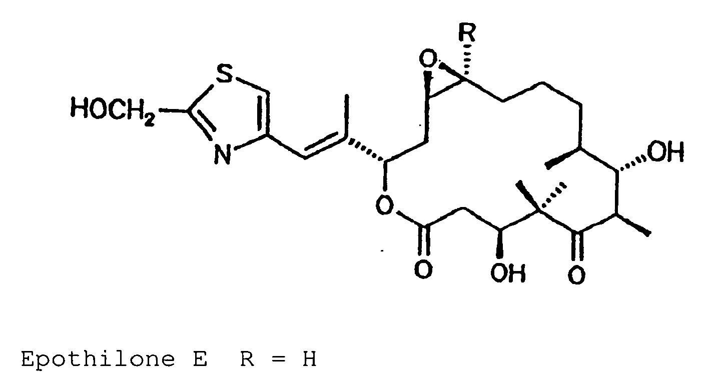

2. Compound of the empirical formula C₂₆H₃₉NO₇S according to claim 1, **characterized by** the following ¹H-NMR spectrum (300 MHz, CDCl₃): delta = 2.38 (2-Hₐ), 2.51 (2-H_{b}), 4.17 (3-H), 3.19 (6-H), 3.74 (7-H), 1.30 - 1.70 (8-H, 9-H₂, 10-H₂, 11-H₂), 2.89 (12-H), 3.00 (13-H), 1.88 (14-Hₐ), 2.07 (14-H_{b}), 5.40 (15-H), 6.57 (17-H), 7.08 (19-H), 4.85 (21-H₂), 1.05 (22-H₃), 1.32 (23-H₃), 1.17 (24-H₃), 0.97 (25-H₃), 2.04 (27-H₃).

3. Compound (epothilone F) of the formula:

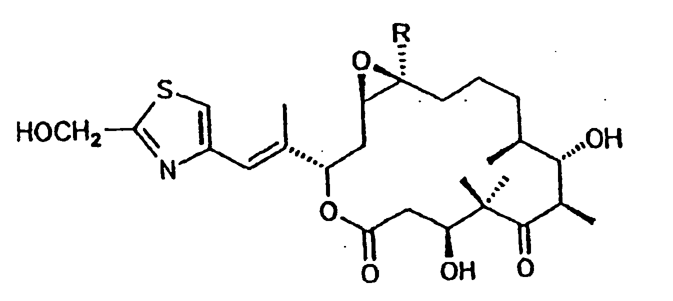

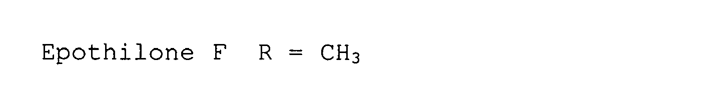

4. Compound of the empirical formula C₂₇H₄₁NO₇S according to claim 3, **characterized by** the following ¹H-NMR spectrum (300 MHz, CDCl₃) : delta = 2.37 (2-Hₐ), 2.52 (2-H_{b}), 4.20 (3-H), 3.27 (6-H), 3.74 (7-H), 1.30 - 1.70 (8-H, 9-H₂, 10-H₂, 11-H₂), 2.78 (13-H), 1.91 (14-H), 2.06 (14-H_{b}), 5.42 (15-H), 6.58 (17-H), 7.10 (19-H), 4.89 (21-H₂), 1.05 (22-H₃), 1.26 (23-H₃), 1.14 (24-H₃), 0.98 (25-H₃), 1.35 (26-H₃), 2.06 (27-H₃).

5. Therapeutical agent, in particular for use as a cytostatic, consisting of one or more of the compounds according to one or more of the preceding claims or consisting of one or more of the compounds according to one or more of the preceding claims in addition to one or more customary carrier(s) and/or diluent(s).

## Revendications

1. Composé (épothilone E) de formule :

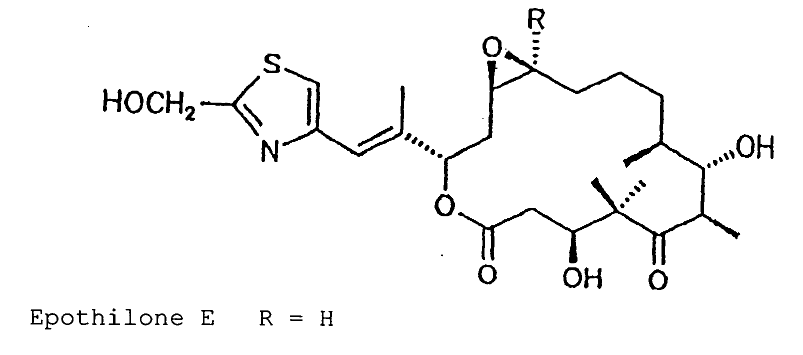

2. Composé de formule brute C₂₆H₃₉NO₇S selon la revendication 1, **caractérisé par** le spectre ¹H-RMN suivant (300 MHz, CDCl₃) : delta = 2,38 (2-Hₐ), 2,51 (2-H_{b}), 4,17 (3-H), 3,19 (6-H), 3,74 (7-H) , 1, 30 - 1,70 (8-H, 9-H₂, 10-H₂, 11-H₂), 2,89 (12-H), 3,00 (13-H), 1,88 (14-Hₐ), 2,07 (14-H_{b}), 5,40 (15-H), 6,57 (17-H), 7,08 (19-H), 4,85 (21-H₂), 1,05 (22-H₃), 1,32 (23-H₃), 1,17 (24-H₃), 0,97 (25-H₃), 2,04 (27-H₃).

3. Composé (épothilone F) de formule :

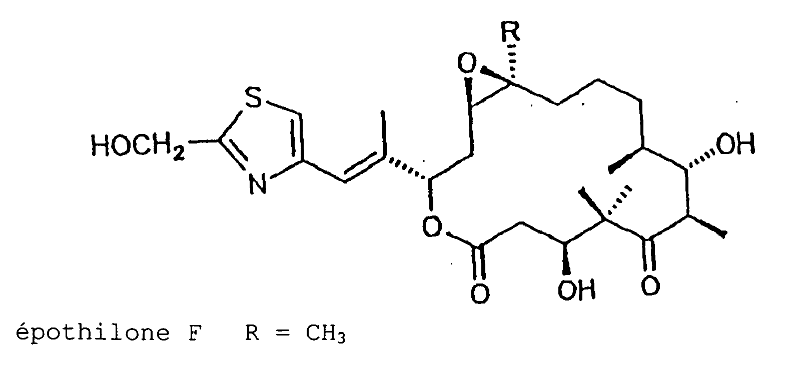

4. Composé de formule brute C₂₇H₄₁NO₇S selon la revendication 3, **caractérisé par** le spectre ¹H-RMN suivant (300 MHz, CDCl₃) : delta = 2,37 (2-Hₐ), 2,52 (2-H_{b}), 4,20 (3-H), 3,27 (6-H), 3,74 (7-H), 1,30 - 1,70 (8-H, 9-H₂, 10-H₂, 11-H₂), 2,78 (13-H), 1,91 (14-H), 2,06 (14-H_{b}), 5,42 (15-H) , 6,58 (17-H) , 7,10 (19-H) , 4, 89 (21-H₂), 1, 05 (22-H₃), 1, 26 (23-H₃), 1,14 (24-H₃), 0,98 (25-H₃), 1,35 (26-H₃), 2,06 (27-H₃)_{.}

5. Agent thérapeutique, en particulier pour la mise en oeuvre en tant que cytostatique, constitué d'un ou de plusieurs composés selon une ou plusieurs des revendications précédentes ou constitué d'un ou de plusieurs composés selon une ou plusieurs des revendications précédentes en plus d'un ou de plusieurs excipients et/ou agents diluants usuels.
